# EUROPÄISCHE PATENTSCHRIFT

(11) **EP 2 282 751 B1**
(45) Veröffentlichungstag und Bekanntmachung des Hinweises auf die Patenterteilung: **09.07.2014**
(21) Anmeldenummer: 08801930.2
(22) Anmeldetag: 09.09.2008
(51) Int. Cl.: A61K 36/16, A61K 36/25, A61K 36/258, A61P 25/24, A61P 25/28, A61P 9/10

(54) **KOMPARTIMENTSPEZIFISCHE PFLANZENEXTRAKTKOMBINATION AUS GINKGO BILOBA- UND GINSENG-EXTRAKT MIT TANDEMWIRKUNG**
COMPARTMENT-SPECIFIC PLANT EXTRACT COMBINATION OF GINKGO BILOBA EXTRACT AND GINSENG EXTRACT HAVING A TANDEM EFFECT
COMBINAISON D'EXTRAITS DE PLANTES POSSÉDANT UNE SPÉCIFICITÉ DE COMPARTIMENT ET CONTENANT UN EXTRAIT DE GINKGO BILOBA ET DE GINSENG EXERÇANT UN EFFET TANDEM

(30) Priorität: 22.04.2008 DE 102008020127
(43) Veröffentlichungstag der Anmeldung: 16.02.2011
(73) Patentinhaber: Merz Pharma GmbH & Co. KGaA, 60318 Frankfurt Main (DE); Beutler, Ralf, 64739 Höchst-Hummetroth (DE); SCHMIDT, Karlheinz, Prof. Dr. Dr., 72810 Gomaringen (DE)
(72) Erfinder: BEUTLER, Rolf, D., 64739 Höchst/Hummetroth (DE); SCHMIDT, Karlheinz, 72810 Gomaringen (DE)
(74) Vertreter: Müller, Claudia
(86) Internationale Anmeldenummer: PCT/EP2008/007357
(87) Internationale Veröffentlichungsnummer: WO 2009/129833

(56) Entgegenhaltungen:
- EP-A- 1 537 874
- WO-A-01/43753
- WO-A-2007/118363
- WO-A-2007/124674
- DE-A1-102006 019 863
- WESNES K A ET AL: "THE MEMORY ENHANCING EFFECTS OF A GINKO BILOBA/PANAX GINSENG COMBINATION IN HEALTHY MIDDLE-AGED VOLUNTEERS" PSYCHOPHARMACOLOGY, SPRINGER VERLAG, BERLIN, DE, Bd. 152, Nr. 4, 1. Januar 2000 (2000-01-01), Seiten 353-361, XP001029198 ISSN: 0033-3158
- DATABASE WPI Week 200347 Thomson Scientific, London, GB; AN 2003-497986 XP002542483 & JP 2003 038140 A (IDEALCOMS KK) 12. Februar 2003 (2003-02-12)
- IWAOKA EMIKO ET AL: "Preventive effect of the Chinese herbal medicine 'Myakuryu' on hypertension and stroke in stroke-prone spontaneously hypertensive rats" CLINICAL AND EXPERIMENTAL PHARMACOLOGY AND PHYSIOLOGY, Bd. 34, Nr. Suppl. 1, November 2007 (2007-11), Seiten S51-S52, XP002542482 ISSN: 0305-1870

## Beschreibung

### Gegenstand der Erfindung

Die vorliegende Erfindung betrifft eine insbesondere (synergistisch) überadditiv wirksame Pflanzenextrakt- Kombination aus Ginkgo und Ginseng, mit optionalem Zusatz an Weißdompulver oder Weißdomextrakt, wobei Ginseng- und Ginkgo-Extrakt in einem Verhältnis von ≤ 1:1 eingesetzt werden. Dadurch kann unerwarteter Weise insbesondere eine verbesserte Prophylaxe, ggf. auch Behandlung, gegenüber einer Alters- oder auch durch sonstige Störungen bedingten Minderung der mentalen Leistung wie Aufmerksamkeit, Konzentration, Gedächtnis erzielt werden. Dies ergibt sich aus einem über- additivem Tandemeffekt durch Schutz neuronaler Zellen und verbesserte Durchblutung einerseits zusammen mit einer verbesserten Energieversorgung und Aktivierung des Gehirnstoffwechsels andererseits, nicht nur im kognitiv- therapeutischen Bereich. Insbesondere können mit der erfindungsgemäßen Kombination vor allem durch Abbauprodukte von Wirksubstanzen hervorgerufene oxidativ- schädliche Einflüsse zurückgedrängt und dadurch ein insbesondere antioxidativer Schutz komplementär auf mehrere Zellkompartimente erstreckt werden.

### Stand der Technik

Pflanzenextrakte in Form von Nahrungsergänzungsmitteln bzw. pflanzlichen Arzneimitteln (Phytopharmaka) werden seit langem zur Verbesserung des Wohlbefindens bzw. der Behandlung unterschiedlichster Befindlichkeitsstörungen eingesetzt, einerseits wegen bekannter Wirkungen und andererseits auch wegen der meist geringen Nebenwirkungen und guten Verträglichkeit. Je nach Einsatzgebiet werden jeweils geeignete Pflanzen gewählt und daraus entsprechende Verabreich-ungsformen hergestellt. Die Gewinnung der wirksamen Bestandteile kann auf verschiedenen Wegen erfolgen. Ein bewährtes Verfahren ist die Extraktion mittels Lösungsmitteln. Dabei kann ein flüssiger Extrakt, ein Spissum oder auch ein Trockenextrakt erhalten werden, je nach Pflanzen und / oder gewünschter nachfolgender Darreichungsform. Da der Wirkstoffgehalt der Heilpflanze selbst je nach Standort, Witterungsbedingungen, Emtezeitpunkt schwanken kann, werden die Verfahren zu ihrer Gewinnung jeweils abgestimmt und das Auszugsverfahren von der Gewinnung der Pflanze bis hin zum gebrauchsfertigen Präparat kontrolliert, um gleichbleibende Qualität der Produkte zu gewährleisten.

In den letzten Jahren haben sich eine Vielzahl bekannter Pflanzenextrakte als Phytopharmakologische Produkte etabliert, wie Arnika, Rosskastanienextrakt, Kamille, Aloe vera, Grüntee, Echinacea, zur Anwendung bei Entzündungen, arteriellen oder venösen Beschwerden, Hautfunktionsstörungen, zur immunologischen Stärkung etc., wobei sowohl eine topische als auch eine enterale Verabreichung möglich sein kann.

Eine weitere seit langem bekannte Pflanze ist Panax ginseng (Koreanischer Ginseng nach C. F. Meyer). Dieser wird als Bestandteil von Tonika eingesetzt und hat eine allgemein stimulierende bzw. vitalisierende Wirkung auf die Gehirnfunktion wie z. B. eine Erhöhung der Leistungsfähigkeit, bei Konzentrationsschwäche oder Stärkung und Kräftigung auch in der Rekonvaleszenz. Auch Weißdorn (Crateagus), ist eine bekannte Heilpflanze, die traditionell zur Unterstützung der Herz-Kreislauf- Funktion und zur Erhöhung der Spannkraft vorgeschlagen wird. Besondere Aufmerksamkeit gilt Ginkgo biloba, welcher in jüngster Zeit häufig als solcher oder in Kombination mit anderen wirksamen Bestandteilen wie Roßkastanie eingesetzt wird. Dabei soll Ginkgo biloba allgemein als Mittel zur Verbesserung der Durchblutung und der Sauerstoffversorgung und daher vor allem zur Behandlung von himorganisch bedingten Störungen, Konzentrationsschwankungen, Gedächtnisschwäche, Stimmungslabilität wirksam verwendet werden können. Hierzu wird normalerweise ein standardisierter Ginkgo- Extrakt aus Blättern des Ginkgo- Baumes mit einem Anteil an als wirksame Substanzgruppe angesehenen Ginkgoflavonglykosiden von meist 24-36% eingesetzt. Ein derartiger Extrakt ist beispielsweise Ginkgo- Extrakt Egb761®, welcher insbesondere nach Schädigung von Nervenzellen, also therapeutisch wirksam sein soll. Es handelt sich um einen Aceton - Extrakt (Tebonin®, Egb 761®) mit einem Droge- Extrakt- Verhältnis von 35-67:1 und 22-27 % Ginkgo-Flavon-(ol)- glykosiden. Im Produkt Ginkobil-N-ratiopharm® wird ein Ginkgo-Extrakt eingesetzt, welcher pro Filmtablette 40 mg Trockenextrakt aus Ginkgo-biloba- Blättern mit einer standardisierten Menge von 8.8 bis 10,8 mg Flavonglykosiden aufweist. Das Mittel soll z. B. bei dementiellem Syndrom und der arteriellen Verschlusskrankheit Verwendung finden. Dazu werden täglich 3x1-2 Tabletten, also 120 bis 240 mg Ginkgo-Extrakt empfohlen.

In der DE 600 09 145 T2 (EP 1 242 105 B1) wird ein Ginkgo- Extrakt zusammen mit Panax ginseng (Wurzel-)-Extrakt zur Steigerung des kognitiven Vermögens bei gesunden Kindern und jungen Erwachsenen unmittelbar vor einer bekannten Bedarfslage wie Prüfungen oder anderen geistigen Anforderungen vorgeschlagen. Dazu wird eine Kombination eingesetzt, worin der Gewichtsanteil an Ginsengextrakt 1,6 mal höher ist als der Gewichtsanteil an Ginkgo- Extrakt. Letzterer soll mit Wasser / Ethanol, oder Mischungen hiervon oder Ölen gewonnen werden können. Auch soll Gincosan® geeignet sein, welches 60 mg Ginkgo- Extrakt GK501 mit 24,5 % Ginkgo-flavonoiden, und 100 mg Panax ginseng C.H. Meyer Extrakt G115 (DEV 5:1) mit 4% Ginsenosiden enthält, Verhältnis Ginseng : Ginkgo: 1,66:1. Es wird vermutet, dass diese Kombination eine Blutplättchen- Aggregations- hemmende Wirkung, oder auch eine immunmodulierende ausüben könnte. Gute Testergebnisse sollen hier mit mindestens mehr als 200 bis 1200 mg Kombinationspräparat, vorzugsweise 320, 640, 960 mg Kombinationspräparat erzielt werden, letztere entsprechend ab 100 % Ginkgo bzw. ab ca. 100 % Ginseng der unteren Monographietagesdosis (120 mg Ginkgo bzw. ca.1 g Ginseng) bzw. bis 150 % der maximalen Monographiedosierung (240 mg Ginkgo, 2000 mg Ginseng), z. B. oral 0,5 bis 6 Stunden vor der Anforderung.

Somit werden spezielle Extrakte bzw. spezielle Kombinationen für ganz spezielle Behandlungen bereitgestellt.

### Aufgabe vorliegender Erfindung

Nach wie vor besteht ein Bedarf an Mitteln, mit denen zuverlässig Alters- oder durch sonstige Störungen bedingte Minderungen der mentalen Leistung insbesondere vorgebeugt und damit auch wirksam begegnet werden können. Wünschenswert wäre dabei, dass der Gesamtkomplex Hirnleistung / Durchblutung / Sauerstoffverwertung angesprochen werden könnte und infolgedessen über die himorganische Leistungserhaltung bzw. Verbesserung auch die arterielle Leistung des Herz- Kreislauf- Systems positiv angesprochen werden könnte.

### Lösung der Aufgabe

Diese Aufgabe wird erfindungsgemäß dadurch gelöst, dass eine Kombination aus Ginkgo biloba - Extrakt und Ginseng Extrakt bereitgestellt wird, mit welcher bei präventiver, insofern auch therapeutischer Anwendung, ein Ginkgo- bedingter neuronaler Zellschutz und dadurch eine verbesserte Gehirndurchblutung zusammen mit einer Ginseng bewirkten, kombinationsbedingt überadditiv verbesserten Energieversorgung der Zellen und damit des Gehimstoffwechsels erzielt werden kann. Dies führt zu einer wirksamen Vorbeugung gegen eine insbesondere Altersbedingte Minderung der mentalen Leistungskraft und lässt sich anhand verbesserter Aufmerksamkeit, Konzentration, Gedächtnisleistung ersehen. Die erfindungsgemäße Kombination weist im Gegensatz zu oben beschriebenen Mitteln einen vor allem Aceton, insbesondere Aceton - Wasser Gingko- biloba- Blätter-Extrakt (trocken), DEV, 40-53:1, vor allem 45-49:1 und insbesondere 50:1, und einen DEV, 3,3-3,8:1, vor allem 3,6 :1 Ginseng, vor allem Panax ginseng-, z. B: Ethanol / oder Ethanol-Wasser - Extrakt auf, wobei das (Gewichts-)Verhältnis von Ginseng Extrakt zu Ginkgo-Extrakt ≤ 1:1 bis z. B. 0,7:1 ist. Überraschender Weise kann durch die erfindungsgemäße Tandem Wirkung insbesondere ein überadditiver Kompartiment- spezifischer antioxidativer Effekt erzielt werden, indem durch jeweilige Abbauprodukte der Wirksubstanzen hervorgerufene oxidativ- schädliche Einflüsse zurückgedrängt und dadurch ein antioxidativer Schutz komplementär auf mehrere Zellkompartimente erstreckt wird, was zu einer unerwarteten Verbesserung der Energiebereitstellung und Ausnutzug des Zellstoffwechsels führt. Dies führt überraschender Weise nicht nur bei der Behandlung, sondern bereits prophylaktisch sowohl zu einer vermehrten Toleranz der Zellen gegenüber Sauerstoffmangelzuständen (Hypoxien) als auch einer beschleunigten Adaption der Nährstoffbedarfslage.

Vorzugsweise beträgt im Gegensatz zu bisherigen Kombinationen das (Gewichts-)Verhältnis von Ginkgoextrakt zu Ginseng- vorzugsweise Panax ginsengExtrakt 1,4:1 bis 1,25:1, vor allem 1,3 :1 bis 1,2 :1, vorteilhafter Weise auch bis 1,1:1 oder auch 1,1:1, in einer weiteren Ausführungsform auch 1:1. Vorteilhafter Weise weist der Ginkgoextrakt 15-30 %, oder auch 17-28 %, besonders 18-27 %, Ginkgoflavonglykoside und der Ginsengextrakt 3-6%, insbesondere 3,5-5 % oder auch 4% Ginsenoside auf.

Eine derartige Kombination mit synergistisch- antioxidativem Komplementär- Tandem- Effekt, worin der Anteil an Ginseng, insbesondere Panax ginseng-Extrakt, gleich dem oder vor allem geringer als der Mengenanteil an Ginkgo biloba Extrakt ist, kann in einer weiteren bevorzugten Ausführungsform noch einen Anteil an Weißdorn (Crataegus) aufweisen wie nachfolgend beschrieben.

Wie erläutert, erfolgt für die jeweiligen Pflanzenextrakte eine jeweils unterschiedliche Wirkung, die bei Kombination zu Synergieeffekten führt und damit eine vorbeugende Wirkung beinhaltet. Der von Ginkgo hervorgerufene neuronale Schutzeffekt im Sinne einer verbesserten Toleranz gegen hypoxische Zustände kann dabei durch Ginseng bei dem angegebenen Mengenverhältnis gesteigert werden, was wiederum zu einer an sich Ginseng bedingten Verbesserung der Energiebereitstellung führt.

Für Ginseng kann dabei weiterhin überraschender Weise auch eine Korrelation mit Superoxid- Dismutase (SOD) angenommen werden, wobei insgesamt eine adaptogene Wirkung (beschleunigte Anpassung der Körperfunktionen an entsprechende Leistungsanforderungen) im Vordergrund steht. Crataegus- Zusatz führt auf unerwartete Weise zu einem weiteren protektiven Effekt im kardiologischen Bereich. Damit kann die insbesondere prophylaktische Wirkung der erfindungsgemäßen Kombination noch verstärkt werden. Dabei kann - ohne auf eine bestimmte Theorie festgelegt zu sein- davon ausgegangen werden, dass sich aufgrund des Tandem- Effekts eine Wirkung auf mehrere Kompartimente der Zellen erstreckt, wobei potentiell schädliche Zwischenprodukte bei der Reaktion der einen Komponente durch die Reaktion mit der/n anderen Komponente/n effektiv entgiftet werden.

### Nähere Erläuterung der Erfindung und bevorzugte Ausführungsformen

Erfindungsgemäß bereitgestellte Kombinationen umfassen Ginkgo biloba (aus Blättern) und Ginseng, vor allem Panax ginseng (vor allem Ginsengwurzel), jeweils in Form von - vorzugsweise Trocken- Extrakten, wobei das (Gewichts-)Verhältnis von Ginkgoextrakt zu Ginsengextrakt 1,4:1, vor allem 1,3:1, insbesondere 1,15:1 oder 1:1, beträgt. Bevorzugt liegt das Droge- Extrakt- Ver-hältnis bei Ginkgo biloba zwischen, 40-53:1, vorzugsweise 50:1. Der Ginkgoextrakt (vor allem Aceton/Wasser 60:40) weist vor allem 15-30, vor allem 18-27%, Ginkgoflavonglykoside, und der Ginseng (insbesondere Wurzel-)- Extrakt, welcher vorzugsweise ein Alkohol, insbesondere Ethanol oder Ethanol / Wasser, z. B. 20:80 bis 60:40 (m/m), vorzugsweise Panax ginseng 60% Ethanol-Wasser - Extrakt ist, 2-6%, vor allem 2,4 bis 5% und insbesondere 3,5 - 4 % Ginsenoside auf.

Besonders bevorzugt sind Kombinationen, worin das (Gewichts)-Verhältnis von Ginkgo- zu Ginsengextrakt 1,4:1 bis 1:0,75, vor allem 1,4:1 bis 1,25:1, und ganz besonders bevorzugt 1,4:1 beträgt.

Weiterhin vorteilhafte Ausführungsformen umfassen Kombinationen, worin im Ginkgko- Extrakt 19-30 %, bevorzugt 18-27% Ginkgoflavonglykoside vorhanden sind.

In einer weiteren vorteilhaften Ausgestaltung vorliegender Erfindung weist die Kombination zusätzlich einen Anteil an Crataegus spec. (Trocken- Pulver), z. B. aus a) Weißdomfrüchten (Beeren) und / -oder , b) Weißdornblättern / Blüten oder Mischungen hiervon, z. B. im Verhältnis 3:1 bis 1:3, vorzugsweise 1,6:1 bis 1:2, insbesondere 1,5 :1 bis 1:1 (bezogen auf Blätter / Blüten im Verhältnis zu Früchten) auf. Die Menge an Crataegus spez. kann 5-40 Gew.%, vorzugsweise 10 bis 35 Gew. %,insbesondere 12 bis 22 Gew.%, bezogen auf das Gesamtgewicht der Kombination, und das Gewichtsverhältnis von Ginkgo biloba zu Crategus spec. (Gesamt)- Extrakt 1: 1 bis 1:1,3, vorzugsweise 1:1 betragen.

### Herstellung der Extrakte

Wie erläutert, wird erfindungsgemäß vorzugsweise ein nicht ethanolischer / öliger Lösungsmittel - Auszug aus Gingko-biloba- Blättern verwendet. Die nach insbesondere kontrolliertem Anbau gewonnenen Blätter können als solche oder getrocknet eingesetzt werden. Vorzugsweise werden sie getrocknet, sodann in üblicher Weise mechanisch gereinigt (durch Sieben), in gattungsgemäßen Drogenmühlen auf die gewünschte Korngröße zerkleinert und anschließend im Lösungsmittel, z. B. Aceton oder vor allem einem Aceton - Wasser Gemisch, z. B. 40:60 bis 70:30 (m/m), insbesondere Aceton : Wasser 60:40 (m/m) extrahiert. Der erhaltene meist dünnflüssige Extrakt kann, ggf. nach weiteren Aufbereitungsschritten z.B. einer schonenden Eindickung bzw. einer anschließenden schonenden Trocknung (z. B. Vakuum) unterworfen werden. Dabei wird ein dickflüssiger Extrakt bzw. ein Pulver erhalten, welches ggf. auf eine gewünschte Korngröße gemahlen werden kann. Das Verhältnis von trockener Droge zu nativem Extrakt entspricht dann vorzugsweise 40-53:1, insbesondere 50:1.

Als weitere Kombinationskomponente wird Ginsengwurzel - Trockenextrakt, erhalten z. B. durch Lösungsmittelextraktion und Aufarbeitung wie oben beschrieben eingesetzt. Als Auszugsmittel wird hier bevorzugt Alkohol oder ein Alkohol/ Wasser- Gemisch, wie Ethanol / Wasser 40:60 bis 60:40, bevorzugt 60:40 Ethanol / Wasser (VN) verwendet. Auch hier erfolgt die Bereitstellung eines Trockenextraktes (Pulver) wie oben beschrieben. Vorzugsweise werden Panax ginseng Wurzeln eingesetzt und mit Ethanol- Wasser 60:40 (V/V) extrahiert. Als Ergebnis wird ein Panax ginseng DEV von 3.3-3.8:1, insbesondere 3.6:1 erhalten. Vor allem bevorzugt wird Ethanol / Wasser, 60:40 (V/V), für einen Ginseng- Wurzel- Extrakt, insbesondere Panax ginseng Wurzel- mit 3-6% Ginsenosiden enthaltend.

Alternativ kann ggf. auch Sibirischer Ginseng Eleuterococcus senticosus - Wurzel- Extrakt, insbesondere ein DEV von 3.3-3,8:1, bevorzugt 3,6 :1-Eleuterococcus senticosus z. B: Ethanol- vor allem Ethanol- Wasser- Extrakt, z. B. 30:70 bis 60:40, bevorzugt 30:70 (VN) Ethanol/Wasser verwendet werden. Das Verhältnis von Ginkgo- Extrakt zu Eleuterococcus senticosus Extrakt kann vorzugsweise 1,4 :1 bis 1,25:1 oder auch 1:1, vorzugsweise 1:1 oder 1,15:1 betragen.

Sofern zusätzlich Weißdom zum Einsatz kommt, so können hierfür vorzugsweise Früchte (Beeren), Blätter und Blüten gewählt werden. Das Ausgangsmaterial wird getrocknet, ggf. auf die gewünschte Komgröße gemahlen und als Pulver erhalten. Wesentliche Inhaltsstoffe sind Flavonoide, Procyanidine. Bevorzugt kann die erfindungsgemäße Kombination 15- 40 Gew. %, insbesondere 25-35 Gew. %, bezogen auf das Gesamtgewicht der Kombination, an Crataegus spez. Pulver aufweisen. Dieses kann insbesondere eine Mischung aus Blättern + Blüten zusammen mit Früchten sein, z. B. im Verhältnis Blätter+ Blüten : Früchten (Beeren) von 1:2 bis 2:1. besonders bevorzugt sind Mischungen aus Blüten + Blättern ( z. B. Verhältnis 1:10 bis 10:1) zusammen mit Beeren im Verhältnis 1:2 (jeweils Pulver wie beschrieben).

Alternativ kann auch ein Crataegus- Extrakt eingesetzt werden. Auszugsmittel ist hier bevorzugt Alkohol oder Alkohol / Wasser-Gemisch, z. B. 35:65 bis 65: 35 %, vorzugsweise 40:60, insbesondere 45:55 % (V/V)). Als Alkohol eignet sich vor allem Ethanol. Das Verfahren gestaltet sich wie oben beschreiben. Nach dem Trocknen werden Pulver- Extrakte erhalten. Diese können vor allem in Mengen von 5-15 Gew. %, bezogen auf die Gesamtmenge der Kombination, eingesetzt werden. Die Pulver- Extrakte können vorzugsweise 1-3, insbesondere 1-2% Flavonoide und 0,4 - 4% Procyanidine enthalten.

Es ist ferner von Vorteil, wenn in erfindungsgemäßen Produkten eine Mischung aus Weißdornfrüchten (Beeren)- (Pulver), Weißdomblättem mit Blüten (Pulver) vorliegt, vorzugsweise im Verhältnis wie oben angegeben.

### Beschreibung der Kombinationsmittel

Die erfindungsgemäßen Kombinationen (Ginkgo-Blätter- Trockenextrakt, Ginseng-, vor allem Panax ginseng- Wurzeltrockenextrakt, ggf. Weißdorn, in Form getrockneter Blätter, Früchte, Blüten- Pulver, ggf. Extrakt- Pulver) können als Nahrungsergänzungsmittel oder insbesondere als (pharmazeutische) Medikation, Phytopharmakon, vor allem zur Prophylaxe und insofern auch therapeutisch vorliegen. Dazu können die Wirkstoffkombinationen mit geeigneten Zusätzen versetzt werden, wie solche, die bekannt sind zur Herstellung von Dragees, Tabletten, Filmtabletten, Kapseln wie z. Sprengmittel, Füllstoffe, Fließmittel, Träger, Tablettierhilfsmittel Siliciumdioxid und -derivate, Cellulose und -derivate, Magnesiumstearat, Lactose, Saccharose, Wachs, Öle wie Sojaöl, Phospholipide, Polyole wie Sorbitol, Stärkepulver, Celluloseester oder Alkansäuren, Cellulosealkylester, Talk, Stearinsäure, Magnesium-stearat, Magnesiumoxid, Natrium- und Calciumsalzen von Phosphor- und Schwefelsäuren, Gelatine, Akaziengummi, Natriumalginat, Glykole Polyvinylpyrrolidon und/oder Polyvinylalkohol und dann tablettiert oder verkapselt ggf. auch mit Lösehilfen wie Zitronensäure, Hydrogencarbonat kombiniert werden. Diese Zusätze und deren erforderliche bzw. gewünschten Mengen sind dem Fachmann allgemein bekannt. Besonders bevorzugte Hilfsstoffe sind neben weiteren Öle wie Sojabohnenöl, Kokosfett und / oder Phospholipide, z. B. aus Sojabohnen, Ei, Gelatine, Glycerol, Polyole wie Sorbitol, Lactose, Saccharose, Phosphate wie Calciumhydrgen-phosphat, Talkum, Farbstoffe, Cellulose und -derivate, Siliciumdioxid und - derivate, Wachse, Stärke. Besonders bevorzugte Hilfsmittel sind neben üblichen Zusätzen Phospholide.

Mit erfindungsgemäßen Kombinationen können bzgl. der Monographietagesdosierung geeignete Wirkungseinheiten bereitet werden. Dazu gehören z. B. Dragees, oder Tabletten / Kapseln. Diese können von beispielsweise je 10 - 30 %, insbesondere 14-29 % Ginkgo; 10-28%, insbesondere 12-24 % Ginseng (Panax ginseng) und ggf. 1,5 bis 6 %, vor allem 2,5 - 5,3 % Crataegus der empfohlenen Monographietagesdosierungen erhalten werden. Derartige Wirkungseinheiten können bevorzugt verabreicht werden, so dass eine Tagesdosis zwischen z. B. 80 -86% (bezogen auf Maximaldosierung) -100-120, z. B. 115% (bezogen auf untere Dosierung), vor allem 86% Ginkgo, bzw. 25 % (obere Dosierung) bis 75 % (untere Dosierung), insbesondere bis 37% (bezogen auf Maximaldosierung) Panax Ginseng sowie ggf. 5 bis 13%, insbesondere 5 bis 10%, vor allem 10 % Crataegus (bzgl. Crataegus, empfohlene Dosierung ca. 1500 mg) erreicht wird. Vorzugsweise werden Wirkungseinheiten (bezogen auf die untere Monographietagesdosierung) von ca. 27-29 % Ginkgo, 11-13 % Ginseng und ggf. 2- 3 % Crataegus bereitet. Dies bedeutet, dass nicht für alle aktiven Bestandteile die nahezu maximale Wirkdosis oder darüber hinaus eingesetzt werden muss. Obgleich die gewählten Extrakte derzeit als unbedenklich und nicht toxisch gelten, ist es dennoch vorteilhaft, wenn insgesamt weniger Wirkstoff erforderlich ist.

### Anwendung / Wirkungsweise

Aufgrund verschiedener Studien hat sich die Erkenntnis verdichtet, dass z. B. oxidative Änderungen sowohl des neuro- geriatrischen als auch kardio- vaskulären Symptomkomplexes an wichtigen Zellstrukturen zu zunächst meist eher uncharakteristischen Allgemeinsymptomen führen können, die man mit dem Begriff "altersbedingte Leistungsminderung" umschreiben kann.

Mit Hilfe der erfindungsgemäßen Kombinationen kann eine Tandemwirkung erzielt werden, indem durch die jeweiligen Extrakte hervorgerufene Wirkungen sich gegenseitig verstärken: so kann angenommen werden, dass Ginkgo- Extrakt zu einem neuronalen Schutz mit verbesserter Durchblutung des Gehirns führt, während durch Ginseng eine dadurch bedingte erhöhte Energiebereitstellung erzielt wird. Überraschender Weise kann durch diese Synergie aus Schutz und Energie eine optimale umfassendere insbesondere prophylaktische Wirkung erreicht werden. Dies wird auch anhand der nachfolgenden Beispiele, insbesondere auch im Nachweistest der Chemilumineszenz ersichtlich. Insgesamt handelt es sich - ohne auf einen Mechanismus festgelegt zu sein- um einen Kompartiment- spezifischen Tandem- Effekt, indem ein unterschiedlicher Schutz auch gegen durch jeweilige Abbauprodukte der Wirksubstanzen hervorgerufene oxidativ- schädliche Einflüsse erzielt werden kann und sich dadurch der antioxidative / neuronale (Schutz)Effekt komplementär auf mehrere Zellkompartimente erstreckt und somit die Energiebereitstellung hierfür verbessert werden kann. Dadurch kann nicht nur ein spezifischer neuronal - mitochondraler Regenerationseffekt, sondern ein allgemein präventiv - therapeutischer Effekt erzielt werden.

Insofern eignen sich erfindungemäße Kombinationen zur präventiven und / bzw. oder therapeutischen Anwendung bzw. zur Herstellung von Mitteln für diese Anwendung, bzw. als Supplemente, bei mentaler Leistungsstörung, z. B. altersbedingt. Altersleistungsstörungen bedeuten im Sinne vorliegender Erfindung: nachlassende geistige Leistungsfähigkeit (himorganische und /oder kapillarbedingte Leistungsstörungen), Konzentrationsmängel, Gedächtnisverlust oder auch infolgedessen nachlassende körperliche Leistungsfähigkeit wie Ermüdbarkeit, Reizbarkeit, depressive Verstimmung, Angst- und Schwindelzustände, arterielle und periphere Durchblutungsstörungen.

Die Wirksamkeit erfindungsgemäßer Kombinationen kann auch in vivo im COM-PASS- Test (Computerised Mental Performance Assessment) anhand der verbesserten Gedächtnisfunktion (Arbeitsgedächtnis, räumliches Gedächtnis) an ausgewählten gesunden Probanden ermittelt werden. Mittels der Analyse von Delta-, Theta-, Alpha- und Beta- Wellen kann nach der Quantitativen Pharmako-Elektro-Enzephologramm- Methode die Veränderung der Himaktivität nach Einnahme erfindungsgemäßer Kombinationen gemessen werden.

### Herstellung der Kombinationsmittel

Die erfindungsgemäßen Kombinationen werden in an sich bekannter Weise hergestellt bzw. verwendet zur Herstellung nachfolgend beschriebener Mittel wie Tabletten, Kapseln (Gelatinekapseln), Dragees, indem man zunächst die Extrakte / Pulver wie oben beschrieben gewinnt und vorzugsweise als Pulver in geeigneter Weise unter Verwendung jeweils geeigneter Zusatzstoffe für die gewünschte orale Verabreichungsform (insbesondere feste orale Form wie Tabletten, Dragees, Kapseln) wie Träger, Füllstoffe, Tablettierhilfsmittel wie genannt, vermischt und homogenisiert. Methoden zur Herstellung derartiger Mittel / Nahrungsergänzungsmittel, Phytopharmaka oder auch pharmazeutischer Mittel sind z. B. in Lehrbüchern beschrieben und allgemein bekannt. Auf diese Weise können pharmazeutische Zubereitungen oder auch Supplemente (Nahrungsergänzungsmittel) wie beschrieben erhalten werden.

### Anwendung

Die erfindungsgemäßen Kombinationen sind insbesondere für die systemische Anwendung bzw. die Herstellung von Mitteln hierfür geeignet wie oben beschrieben konzipiert. Dabei sind orale Verabreichungsformen wie Gelatinekapseln, z. B. Weichgelatinekapsel, sowie Tabletten, Dragees geeignet.

Zur Herstellung von Kapseln oder Tabletten werden hierfür gebräuchliche Hilfsstoffe wie Lactose, Saccharose, Sorbit, Talkum, Öle, Soja, Stearinsäure, Magnesium-stearat, Gummi arabicum, Kartoffelstärke, Gelatine, PVP, Glycerin, Hydroxypropyl-methylcellulose, Maltodextrin, Konservierungsmittel sowie übliche Materialien für Überzüge wie PEG 6000, Maisstärke, Zucker Talkum, Farbstoffe in an sich bekannter Weise eingesetzt. Wenn per os verabreicht wird, kann die beschriebene Zusammensetzung z. B. vermischt werden mit Lactose, Saccharose, Stärkepulver, Celluloseester oder Alkansäuren, Cellulosealkylester, Talk, Stearinsäure, Magnesiumstearat, Magnesiumoxid, Natrium- und Calciumsalzen von Phosphor- und Schwefelsäuren, Gelatine, Akaziengummi, Natriumalginat, Glykole, Polyvinylpyrrolidon und/oder Polyvinylalkohol und dann tablettiert oder verkapselt werden.

Die Zusatzstoffe (s. auch oben) und Tablettierungsverfahren sind allgemein bekannt und z.B. im aktuellen Europäischen Arzneibuch beschrieben.

Insbesondere können die (Mittel der) Kombinationen präventiv, und insofern auch / oder therapeutisch bei mentaler Leistungsminderungen oder -störung, z. B. Altersbedingt angewendet werden. Altersleistungsminderungen oder -störungen bedeuten im Sinne vorliegender Erfindung: nachlassende geistige Leistungsfähigkeit (hirnorganische und /oder kapillarbedingte Leistungsstörungen), Konzentrationsmängel, Gedächtnisleistungsminderung oder auch infolgedessen nachlassende körperliche Leistungsfähigkeit wie Ermüdbarkeit, Reizbarkeit, depressive Verstimmung, Angst- und Schwindelzustände, arterielle oder periphere Durchblutungsstörungen.

Die Menge des verabreichten Mittels und der Dosierungsplan zur Prävention oder Behandlung eines wie oben beschriebenen Zustandes mit den erfindungsgemäßen Kombinationen kann (z. B. bei einer medizinischen Indikation) von einer Vielzahl von Faktoren abhängen, einschließlich dem Alter, Gewicht, Geschlecht und Zustand des Empfängers, der Verabreichungsroute und kann deshalb weitestgehend schwanken. Die erforderliche oder gewünschte Dosis kann auf ein- oder mehrere Male täglich, insbesondere 1 bis 3 mal verteilt werden.

Bei der Verabreichung als Supplement werden hierfür allgemein übliche Mengen vorgeschlagen, welche auf ein- bis mehrmals, insbesondere 1 bis 3 mal oder 1 bis 2 mal täglich verteilt werden können. Diese können in fester Form, z. B. als Tablette, Kapsel, Dragee, vorliegen. Besonders bevorzugt werden Mengen verabreicht, mit denen die eingangs beschriebenen Monographie- Tagesdosisrationen erreicht werden.

### Beispiele

Die Erfindung wird anhand der nachfolgenden Produktbeispiele 1-5 sowie in Beispiel 6 die Wirkungsnachweise näher erläutert.

### A. Produktbeispiele

### Beispiel 1 (Referenzbeispiel)

Es werden 36 mg eines Ginkgo- Blätter- Pulver- Extrakts, Auszugsmittel Aceton / Wasser 60:40 (m/m), wie oben beschrieben, DEV: 38-57:1, enthaltend 18-27% Ginkgoflavonglykoside, mit 32,4 mg Panax ginsengwurzel- Trockenextrakt, Auszugsmittel Ethanol / Wasser (60:40, V/V), DEV: 2,6-3,5:1, enthaltend 3-5% Ginsenoide, mit Siliciumdioxid, Maisstärke, Gummi arabicum, Calciumstearat, Hydriertes Baumwollsamenöl, Saccharose, Farbstoffen (E 104, E 132), Cellulose in geeigneten Mengen gemischt und zu Dragees verarbeitet, Überzugsmittel: Eudragit E.

### Beispiel 2 (Referenzbeispiel)

Eine Weichgelatinekapsel wurde hergestellt aus: Soja- Phospholipiden, Sojaöl, Gelatine, Glycerin, Glycerol, Kokosfett, Lactose, Mannitol, Wasser, 34 mg Ginkgo-Extrakt- Pulver (Aceton / Wasser 60:40) wie oben beschrieben, DEV 45-67:1 sowie 35,4 mg Panax ginseng- Wurzel- Trocken- Extrakt (Ethanol / Wasser 60:40, DEV 3-4:1).

### Beispiel 3

Ein Dragee wurde hergestellt aus Lactose, Siliciumdioxid, hochdispers, Calcium-Natriumcarbonat, Talkum, Cellulose, Gummi arabicum, Soja- Öl, Titandioxid, Farbstoff E 132, Überzugsmittel Eudragit E sowie 32,4 mg Ginkgo- Extrakt (Auszug Aceton- Wasser 60:40 m/m), DEV 50:1, wie oben beschrieben, 23,2 mg Panax ginseng- Extrakt (Auszug Ethanol / Wasser 60:40,V/V), 35 mg Weißdorn (1:1 Mischung Weißdornfrüchte (Beeren) /, Weißdomblätter und -blüten, jeweils Trockenpulver.

### Beispiel 4

Es werden 36 mg eines Ginkgo- Blätter- Pulver Extraktes, gemäß Beispiel 1, Auszugsmittel Aceton / Wasser 60:40, (m/m) wie oben beschrieben, DEV: 50:1 enthaltend 18-27% Ginkgoflavonglykoside, mit 32,7 mg Panax ginsengwurzel-Trockenextrakt, gemäß Beispiel 1, Auszugsmittel Ethanol / Wasser, 60%, DEV: 3,8:1, enthaltend 3-5% Ginsenoide, 35 mg Weißdorn (2:1 Mischung Weißdornfrüchte (Beeren) /, Weißdomblätter und -blüten, jeweils Trockenpulver, mit Siliciumdioxid, Maisstärke, Gummi arabicum, Calciumstearat, Hydriertes Baumwollsamenöl, Saccharose, Farbstoffen (E 104, E 132), Cellulose in geeigneten Mengen gemischt und zu Dragees verarbeitet, Überzugsmittel: Eudragit E.

### Beispiel 5

Es werden 36 mg eines Ginkgo biloba- Blätter- Pulver- Extraktes, Auszugsmittel Aceton / Wasser 60:40 (m/m), wie oben beschrieben, DEV: 50:1 enthaltend 18-27% Ginkgoflavonglykoside, mit 34,4 mg Eleuterococcus senticosus Wurzel- Trockenextrakt, Auszugsmittel Ethanol / Wasser, 30:70(VN), DEV: 3,8:1, enthaltend 3-5% Ginsenoside, mit hochdispersem Siliciumdioxid, Hypromellose, Lactose-Monohydrat, Macrogol 400, Magnesiumstearat, Talkum, Eisen(III)-oxid E 172 Titrandioxid E 171, Sojabohnenöl, Gelatine, Gelbes Wachs, Sorbitol in geeigneten Mengen gemischt und zu Dragees verarbeitet, Überzugsmittel: Eudragit E.

### B. Wirkungsnachweise

### Beispiel 6

Die mit nachlassender Gedächtnisfunktion im Zusammenhang stehende antioxidative Wirkung wurde im Chemilumineszenz- Assay ermittelt. Diese kann als direktes Maß für einen aktiven umfassenden Zellschutz und damit als Maß für eine prophylaktische Wirksamkeit herangezogen werden, wenn mehrere Teile (Kompartimentspezifisch) erfasst werden.

### Material und Methoden

Für die Untersuchungen zur komplementär antioxidativen Wirkung einer erfindungsgemäßen Kombination aus Ginseng, Ginkgo bzw. und Crataegus wurde die Chemilumineszenz des Vollblutes nach Stimulation mit opsonisiertem Zymosan herangezogen. Bei dieser Methode vermeidet man unerwünschte Beeinflussungen der Zellfunktion, wie sie bei der Arbeit mit isolierten Granulocyten auftreten können, sodass ein gut standardisiertes Mess-System zur Verfügung steht. Als Verstärker wurde Luminol verwendet, die Messungen erfolgten auf einem Biolumaten LB 953.

Zur Messung wurden 0,1 ml Vollblut mit 0,4 ml phosphatgepufferter Saline versetzt, die 0,1% Albumin und 0,1% D-Glucose enthielt. Als Starter wurden 0,2 ml doppelt konzentriertes opsonisiertes Zymosan verwendet. Mit jedem Ansatz wurden über einen Messzeitraum von 20 Minuten 50 Messungen durchgeführt. Jede Einzelmessung dauerte 1,5 Sekunden. Die Messtemperatur betrug 37°C.

Für die verschiedenen Testreihen wurden Kontrollmessungen durchgeführt, und die verschiedenen Prüfsubstanzen eingesetzt.

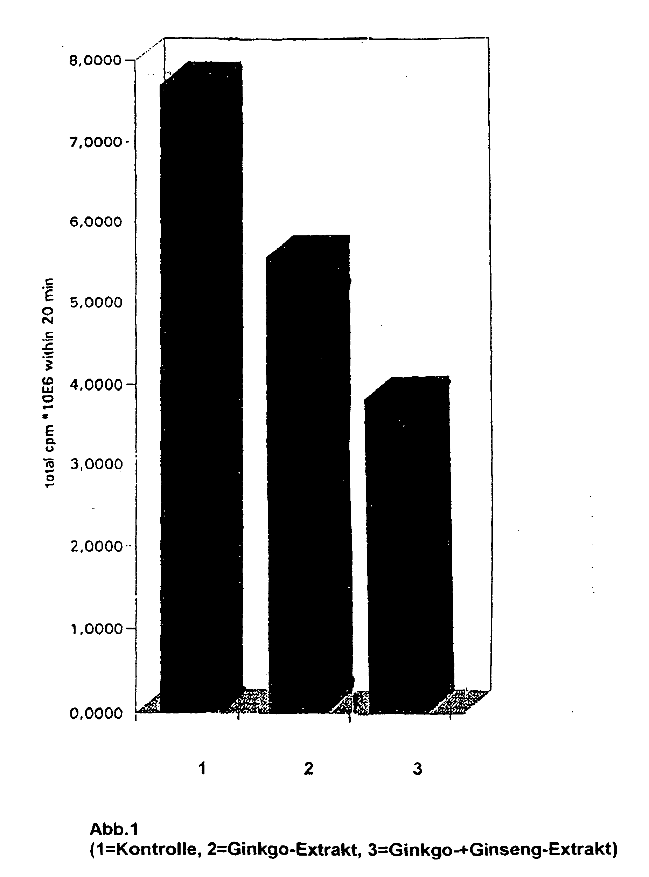

Hierzu wurden 500 mg der jeweiligen Extraktpulver bzw. - Mischung (Ginkgo biloba - Extrakt - Auszug gemäß Beispiel 1, Ginkgo- Extraktpulver + Ginsengwurzelextrakt gemäß Beispiel 1, jedoch Ginkgo jeweils 1:10 verdünnt, Verhältnis Ginseng / Ginkgo wie in Beispiel 1) in 50 ml phosphatgepufferter Saline bei 37° C 20 Minuten gerührt. 0,05 ml des erhaltenen Extraktes wurden zum Vollblutansatz hinzugegeben und bis zum Start der Messung 10 Minuten inkubiert. Die "counts per minute" wurden während des jeweiligen Messzeitraumes registriert und in Abhängigkeit von der Zeit aufgezeichnet. In Abbildung 1 ist das Ergebnis als Kumulierte Chemilumineszenz AUC (Area under the curve) aufgezeichnet, wobei links die Kontrollmessung aufgezeigt ist. Hieraus wird eine verstärkte Hemmung und damit ein erhöhter antioxidativer Effekt für die Kombination aus Ginkgo + Ginseng ersichtlich.

Überraschender Weise zeigt sich, dass z. B. Einzelwirkung von Ginkgo nicht nur beibehalten wird, sondern darüber hinaus verstärkt auftritt und es daher additiv - synergistisch zu einem Zellschutz in verschiedenen Kompartimenten kommt, da die einzelnen antioxidativen Wirkungen unterschiedlich sind und in der erfindungsgemäßen Kombination im angegebenen Mengenverhältnis wie geschildert verstärkt zusammenwirken (Tandemeffekt). Dies lässt sich ohne auf einen Mechanismus festgelegt zu sein, wie folgt erläutern: durch Ginkgo-Extrakt wird ein neuronaler, insbesondere antioxidativ- neuronaler Schutz gegenüber Lipidoxidation mit dadurch bedingter verbesserter Durchblutung erzielt. Dieser Schutz kann aufgrund der Zugabe von Ginseng- Extrakt eine zusätzliche, durch eine dadurch erzielte mehr im Rahmen eines Lipid- Peroxidation- Schutzeffekts liegende neuroprotektive Wirkungsverstärkung erhalten, was umgekehrt wiederum eine synergistisch verbesserte Energiebereitstellung bedingt (Kompartimentspezischer Tandemeffekt).

Überraschenderweise können zusätzlich, insbesondere durch die Ginseng bedingte Anregung der Bildung von Superoxid- Dismutase (SOD) wichtige Metabolite des Sauerstoffs katalytisch, d.h. mit einer hohen Reaktionskinetik abgefangen werden, was zu einer weiteren kompartiment - spezifisch - synergistischen Schutzwirkung im Sinne des beschriebenen Tandemeffekts führt.

Dies beinhaltet insgesamt eine prophylaktive Wirkung gegenüber altersbedingten mentalen Leistungsminderungen bzw. -störungen.

## Patentansprüche

1. Kompartimentspezifisch wirksame Kombination aus Ginkgo biloba- und Ginseng- Extrakten, **dadurch gekennzeichnet, dass** Ginkgo in Form eines Aceton-Wasser Extraktes mit einem DEV von 40-53:1, und Ginseng als Ethanol- Wasser Extrakt mit einem DEV von 3,3-3,8:1 vorliegen und das (Gewichts-)Verhältnis von Ginsengextrakt zu Ginkgoextrakt ≤ 1:1 beträgt.

2. Kombination gemäß Anspruch 1, **dadurch gekennzeichnet, dass** ein Ethanol /Wasser 60:40 (VN)- Panax ginseng- Wurzel-Extrakt, und ein Aceton- Wasser, 60:40 (m/m) Ginkgo- biloba- Blätter- Extrakt vorliegen .

3. Kombination gemäß einem der Ansprüche 1 oder 2 zum präventiven und/oder therapeutischen Kompartimentspezifischen Tandem - Schutz bei mentalen Leistungsminderungen oder -störungen.

4. Kombination zur Verwendung gemäß Anspruch 3, **dadurch gekennzeichnet, dass** die mentalen Leistungsminderungen/-störungen altersbedingte Leistungsminderungen- oder -Störungen sind.

5. Kombination zur Verwendung gemäß Anspruch 3, **dadurch gekennzeichnet, dass** die mentale Leistungsminderung/-störung Ermüdbarkeit, ist.

6. Kombination oder Kombination zur Verwendung gemäß einem der Ansprüche 1 bis 5, **dadurch gekennzeichnet, dass** weiterhin ein Anteil an Crataegus spec. Pulver, ausgewählt aus Weißdornfrüchten (-beeren), Weißdornblättern, Blüten- Trockenpulver oder Mischungen hiervon, enthalten ist.

7. Kombination oder Kombination zur Verwendung gemäß einem der Ansprüche 1 bis 6, **dadurch gekennzeichnet, dass** sie weiterhin neben üblichen Zusätzen / Hilfsmitteln ein oder mehrere Phospholipide aufweist.

8. Kombination oder Kombination zur Verwendung gemäß einem der Ansprüche 1 bis 7, **dadurch gekennzeichnet, dass** sie in Form einer Tablette, eines Dragees oder einer Gelatinekapsel vorliegt.

9. Kombination oder Kombination zur Verwendung gemäß einem der Ansprüche 1 bis 8, **dadurch gekennzeichnet, dass** sie in Form eines oral verabreichbaren Nahrungsergänzungsmittels oder einer (pharmazeutisch zulässigen) prophylaktischen oder therapeutischen Zubereitung (Arzneimittel) vorliegt.

10. Verwendung einer Kombination aus Ginkgo biloba - Extrakt, Auszugsmittel Aceton oder Aceton-Wasser mit einem DEV von 40-53:1, und Ginseng-Wurzel-Extrakt mit DEV von 3,3-3,8:1, Auszugsmittel Ethanol oder Ethanol/Wasser, zur Herstellung eines Mittels zum präventiven und/oder therapeutischen Kompartment-spezifischen Tandem-Schutz bei mentalen Leistungsminderungen oder -störungen als Phytopharmakon oder Nahrungsergänzungsmittel, wobei das (Gewichts-)Verhältnis von Ginseng-Extrakt zu Ginkgoextrakt ≤ 1:1 beträgt.

11. Verwendung gemäß Anspruch 10, **dadurch gekennzeichnet, dass** ein Ethanol/ Wasser 60:40 (V/V)- Panax ginseng- Wurzel-Extrakt, und ein Aceton/Wasser, 60:40 (m/m) Ginkgo- biloba- Blätter- Extrakt vorliegen.

12. Verwendung gemäß einem der Ansprüche 10 oder 11, **dadurch gekennzeichnet, dass** die Kombination neben üblichen Zusätzen/Hilfsmitteln ein oder mehrere Phospholipide aufweist.

13. Verwendung nach einem der Ansprüche 10 oder 12 zur oralen Anwendung in Form eines Dragees, einer Tablette oder eine Gelatinekapsel.

14. Verwendung nach einem der Ansprüche 10 bis 13 zum Kompartiment spezifischen antioxidativen Tandem -Schutz bei der Prävention oder Behandlung von Hirnleistungsstörungen, Ermüdbarkeit, Reizbarkeit, depressiver Verstimmung, Angst- und Schwindelzuständen, arteriellen oder peripheren Durchblutungsstörungen.

## Claims

1. Compartment-specifically effective combination of ginkgo biloba- and ginseng extracts, **characterised in that** ginkgo is present in the form of an acetone/water extract having a DER of 40-53:1, and ginseng is present as an ethanol/water extract having a DER of 3.3-3.8:1, and the (weight) ratio of ginseng extract to ginkgo extract is ≤ 1:1.

2. Combination according to claim 1, **characterised in that** an ethanol/water 60:40 (v/v) Panax ginseng root extract and an acetone/water 60:40 (m/m) ginkgo biloba leaf extract are present.

3. Combination according to either claim 1 or claim 2 for a preventive and/or therapeutic compartment-specific tandem protection in cases of reductions in and problems with mental performance.

4. Combination for use according to claim 3, **characterised in that** the reductions in and problems with mental performance are age-related reductions in and problems with performance.

5. Combination for use according to claim 3, **characterised in that** the reduction in and problem with mental performance is fatiguability.

6. Combination or combination for use according to any of claims 1 to 5, **characterised in that** a proportion of Crataegus spec. powder, selected from hawthorn fruits (berries), hawthorn leaves, dry powder made from flowers, or mixtures thereof, is furthermore contained therein.

7. Combination or combination for use according to any of claims 1 to 6, **characterised in that** it furthermore contains one or more phospholipids, in addition to standard additives/ancillary substances.

8. Combination or combination for use according to any of claims 1 to 7, **characterised in that** it is present in the form of a tablet, a dragee, or a gelatin capsule.

9. Combination or combination for use according to any of claims 1 to 8, **characterised in that** it is present in the form of a nutritional supplement, which can be administered orally, or a (pharmaceutically acceptable) prophylactic or therapeutic preparation (medication).

10. Use of a combination of ginkgo biloba extract, extractant acetone or acetone/water having a DER of 40-53:1, and ginseng root extract having a DER of 3.3-3.8:1, extractant ethanol or ethanol/water, for the production of a substance for the preventive and/or therapeutic compartment-specific tandem protection in cases of reductions in and problems with mental performance, as a phytopharmaceutical or nutritional supplement, wherein the (weight) ratio of ginseng extract to ginkgo extract is ≤ 1:1.

11. Use according to claim 10, **characterised in that** an ethanol/water 60:40 (v/v) Panax ginseng root extract and an acetone/water 60:40 (m/m) ginkgo biloba leaf extract are present.

12. Use according to either claim 10 or claim 11, **characterised in that** the combination contains one or more phospholipids in addition to standard additives/ancillary substances.

13. Use according to either claim 10 or claim 12, for oral administration in the form of a dragee, a tablet, or a gelatin capsule.

14. Use according to any of claims 10 to 13, for compartment-specific antioxidant tandem protection in the prevention or treatment of brain performance problems, fatiguability, irritability, depressive mood, anxiety and dizziness, arterial or peripheral circulation problems.

## Revendications

1. Combinaison d'extraits de Ginkgo biloba et de ginseng ayant une efficacité spécifique à certains compartiments, **caractérisée en ce que** le ginkgo se présente sous forme d'un extrait à l'acétone / eau ayant un rapport drogue / extrait compris entre 40 - 53 : 1, et le ginseng se présentant sous forme d'un extrait à l'eau / éthanol ayant un rapport drogue / extrait compris entre 3,3-3,8 : 1, le rapport (pondéral) entre l'extrait de ginseng et l'extrait de ginkgo étant ≤ 1 : 1.

2. Combinaison selon la revendication 1, **caractérisée en ce qu'**elle se présente sous forme d'un extrait de la racine de Panax ginseng réalisé à l'éthanol / eau 60 : 40 (v/v) et d'un extrait des feuilles Ginkgo biloba réalisé à l'acétone / eau 60 : 40 (m/m).

3. Combinaison selon l'une des revendications 1 ou 2 destinée à exercer un double effet protecteur spécifique à certains compartiments, pour ainsi prévenir et/ou traiter des diminutions ou perturbations des capacités intellectuelles.

4. Combinaison destinée à une utilisation selon la revendication 3, **caractérisée en ce que** lesdites diminutions ou perturbations des capacités intellectuelles correspondent à des diminutions ou perturbations des capacités intellectuelles liées à l'âge.

5. Combinaison destinée à une utilisation selon la revendication 3, **caractérisée en ce que** ladite diminution ou perturbation des capacités intellectuelles correspond à une propension à la fatigue.

6. Combinaison ou combinaison destinée à une utilisation selon l'une des revendications 1 à 5, **caractérisée en ce qu'**elle contient en outre une proportion de Crataegus spec. sous une forme pulvérulente choisie parmi les fruits (cenelles) d'aubépine, les feuilles d'aubépine, les poudres de fleurs séchées ou leurs mélanges.

7. Combinaison ou combinaison destinée à une utilisation selon l'une des revendications 1 à 6, **caractérisée en ce qu'**elle comporte, outre les additifs / adjuvants usuels, un ou plusieurs phospholipides.

8. Combinaison ou combinaison destinée à une utilisation selon l'une des revendications 1 à 7, **caractérisée en ce qu'**elle se présente sous forme d'un comprimé, d'une dragée ou d'une gélule.

9. Combinaison ou combinaison destinée à une utilisation selon l'une des revendications 1 à 8, **caractérisée en ce qu'**elle se présente sous forme d'un complément alimentaire pouvant être administré par la voie orale ou d'une préparation (pharmaceutiquement acceptable) à effet prophylactique ou thérapeutique (médicament).

10. Utilisation d'une combinaison entre un extrait de Ginkgo biloba, le milieu d'extraction étant l'acétone ou l'acétone / eau ayant un rapport drogue / extrait compris entre 40- 53 : 1, et un extrait de racine de ginseng ayant un rapport drogue / extrait compris entre 3,3 - 3,8 : 1, le milieu d'extraction étant l'éthanol ou l'éthanol / eau, pour préparer un agent lequel est destiné à exercer un double effet protecteur spécifique à certains compartiments et lequel permet ainsi de prévenir et/ou traiter des diminutions ou perturbations des capacités intellectuelles, ledit agent se présentant sous forme d'une préparation à base de plantes médicinales ou d'un complément alimentaire, le rapport (pondéral) entre l'extrait de ginseng et l'extrait de ginkgo étant ≤ 1 : 1.

11. Utilisation selon la revendication 10, **caractérisée en ce qu'**elle se présente sous forme d'un extrait de racine de Panax ginseng réalisé à l'éthanol / eau 60 : 40 (v/v) et d'un extrait des feuilles Ginkgo biloba réalisé à l'acétone / eau 60 : 40 (m/m).

12. Utilisation selon l'une des revendications 10 ou 11, **caractérisée en ce que** ladite combinaison comporte, outre les additifs / adjuvants usuels, un ou plusieurs phospholipides.

13. Utilisation selon l'une des revendications 10 ou 12, destinée à une application orale sous forme d'une dragée, d'un comprimé ou d'une gélule.

14. Utilisation selon l'une des revendications 10 à 13, destinée à exercer un double effet protecteur antioxydant spécifique à certains compartiments, pour ainsi prévenir et/ou traiter des perturbations des capacités cérébrales, la propension à la fatigue, l'irritabilité, les états dépressifs, des états d'angoisse et de vertige, des troubles de la circulation artérielle ou périphérique.
